(19) Europäisches Patentamt
European Patent Office
Office européen des brevets

(11) Veröffentlichungsnummer: **0 124 742**
**B1**

(12) # EUROPÄISCHE PATENTSCHRIFT

(45) Veröffentlichungstag der Patentschrift: **31.08.88**

(51) Int. Cl.⁴: **C 07 D 211/90** // C07C79/46

(21) Anmeldenummer: **84103304.6**

(22) Anmeldetag: **26.03.84**

(54) **Verfahren zur Herstellung von symmetrischen 1,4-Dihydropyridindicarbonsäureestern.**

(30) Priorität: **05.04.83 DE 3312216**

(43) Veröffentlichungstag der Anmeldung: **14.11.84 Patentblatt 84/46**

(45) Bekanntmachung des Hinweises auf die Patenterteilung: **31.08.88 Patentblatt 88/35**

(84) Benannte Vertragsstaaten: **AT BE CH DE FR GB IT LI LU NL SE**

(56) Entgegenhaltungen:
**DE-A-1 670 824**
**DE-A-1 792 764**
**DE-A-2 335 466**
**DE-A-2 549 568**
**US-A-4 370 334**

**ORGANIC Reactions, Band 15, 1967, Seiten 204-206, 265, 271, 478; J. WILEY & SONS, INC., New York, London, Sydney**

(73) Patentinhaber: **BAYER AG, Konzernverwaltung RP Patentabteilung, D-5090 Leverkusen 1 Bayerwerk (DE)**

(72) Erfinder: **Teller, Werner, Dr., In den Birken 79, D-5600 Wuppertal 1 (DE)**
Erfinder: **Koebernick, Wolfgang, Dr., Claudiusweg 3, D-5600 Wuppertal 1 (DE)**
Erfinder: **Haaf, Arthur, Dr., Georg- Arends- Weg 25, D-5600 Wuppertal 21 (DE)**
Erfinder: **Naab, Paul, Dr., Schenkstrasse 32b, D-5600 Wuppertal 21 (DE)**
Erfinder: **Preiss, Michael, Dr., Egenstrasse 21, D-5600 Wuppertal 1 (DE)**

**EP 0 124 742 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft ein chemisch eigenartiges Verfahren zur Herstellung von bekannten symmetrischen 1,4-Dihydropyridindicarbonsäureestern.

Mehrere Verfahren zu ihrer Herstellung sind bereits bekannt.

Kirchner, Ber. 25, 2786 (1892) beschreibt die Umsetzung von Aldehyden mit 3-Ketocarbonsäureestern und Ammoniak gemäß folgendem Reaktionsschema:

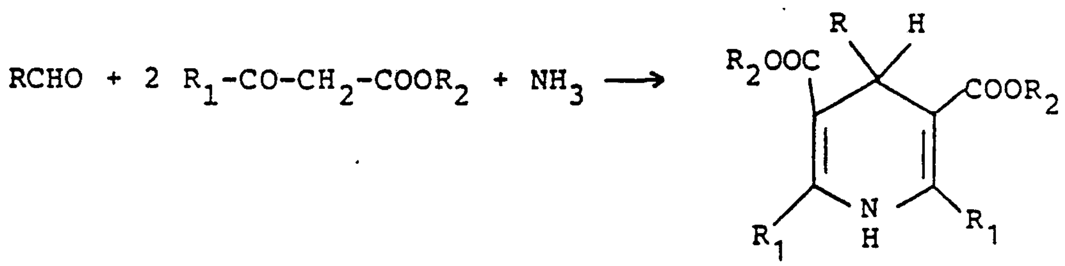

Fox et al. J. Org. Chem. 16, 1259 (1951) erwähnt die Umsetzung von Aldehyden mit 3-Ketocarbonsäureestern und Enaminocarbonsäureestern gemäß folgendem Reaktionsschema:

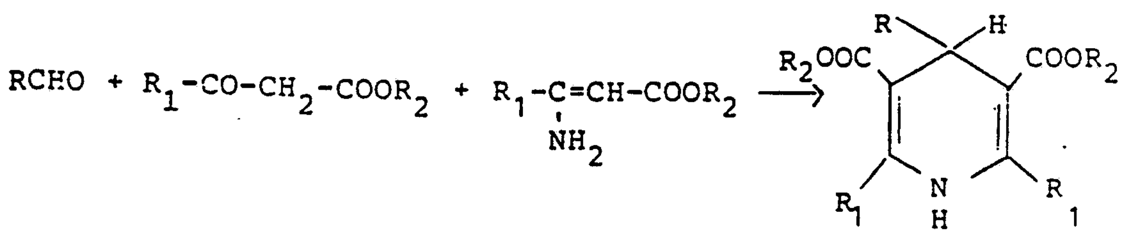

Knoevenagel, Ber. 31, 743 (1898) beschreibt die Umsetzung von Yliden-3-ketocarbonsäureestern mit Enamincarbonsäureestern gemäß folgendem Reaktionsschema:

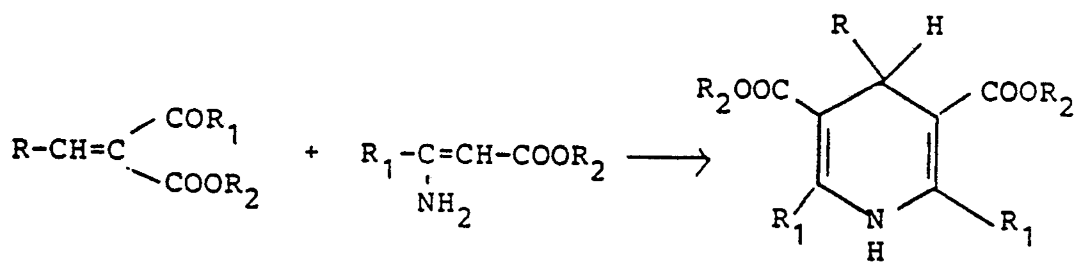

Da sowohl Aldehyde als auch Ammoniak mit Ketocarbonsäureestern reagieren, ist anzunehmen, daß auch bei den beiden erstgenannten Verfahren zunächst die Ylidenverbindung und die Enaminverbindung gebildet werden.

Die Nachteile der geschilderten Verfahren sind darin zu sehen, daß die Ylidenverbindungen nur schwierig in reiner Form zu isolieren sind und daß die 1,4-Dihydropyridindicarbonsäureester daher noch Verunreinigungen enthalten, die durch Reinigungsverfahren nur sehr schwer zu entfernen sind.

Im Hinblick auf die Verwendung der 1,4-Dihydropyridine als Arzneimittel besteht ein Bedürfnis, diese Verbindungen mit einen hohen Reinheitsgrad zur Verfügung zu stellen. So konnten beispielsweise bei der Herstellung von 4-(2'-Nitrophenyl)-2,6-dimethyl-3,5-dicarbethoxy-1,4-dihydropyridin nach US-3 485 847 sieben Nebenprodukte durch Dünnschichtchromatographie festgestellt werden.

In Organic Reactions Bd. 15 (1967), Seiten 204-206, 265, 271 werden bereits Kondensationsreaktionen mit Aldehyden und β-Ketoverbindungen beschrieben, wobei teilweise auch die Verwendung von Katalysatoren erwähnt wird. Diese Publikation gibt jedoch keinen Hinweis darauf, daß durch die Verwendung von Piperidinacetat als Katalysator die Ylidenverbindungen in unerwartet hoher Reinheit und excellenten Ausbeuten entstehen. In den deutschen Offenlegungsschriften Nr. 2 549 568, Nr. 2 335 466, Nr. 1 792 764 und Nr. 1 670 824 wird ebenfalls die Herstellung von Dihydropyridinen beschrieben entweder als 3-Komponentenreaktion aus Aldehyden, β-Ketocarbonsäureestern und Ammoniak bzw. Aminen, oder auch als 2-Komponentenreaktion durch Umsetzung von Ylidenverbindungen mit Enaminverbindungen. In keiner dieser Anmeldungen ist die Verwendung des erfindungsgemäßen Katalysators und sein unerwartet positiver Einfluß auf Reinheit und Ausbeute der Endprodukte erwähnt. Das Gleiche gilt für das US-Patent Nr. 4 370 334. Bei Kenntnis dieses Standes der Technik war nicht zu erwarten, daß durch die Verwendung von Piperidinacetat als Katalysator sowohl die Ylidenverbindungen als auch die daraus herstellbaren Dihydropyridine in so hohen

Ausbeuten und in solcher Reinheit erhalten werden.

Die Erfindung betrifft somit ein Verfahren zur Herstellung von symmetrischen 1,4-Dihydropyridinen der Formel I

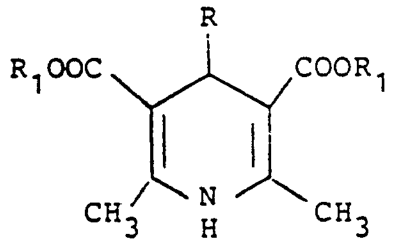

(I)

in der

R einen Phenylrest darstellt, der gegebenenfalls ein- oder zweifach substituiert ist, durch Nitro und/oder Chlor und

$R_1$ einen $C_1$-$C_4$-Alkylrest darstellt, der gegebenenfalls durch eine $C_1$-$C_4$-Alkoxygruppe substituiert ist, durch Umsetzung einer Ylidenverbindungen der Formel II

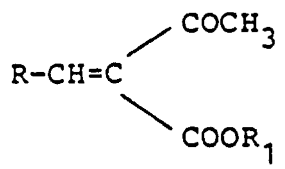

(II)

mit einer Enaminverbindung der Formel III

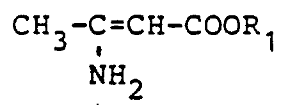

(III)

dadurch gekennzeichnet, daß man die Ylidenverbindung der Formel II durch Reaktion eines Ketocarbonsäureesters der Formel IV

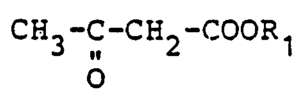

(IV)

mit einem Aldehyd der Formel RCHO in einem Lösungsmittel in Gegenwart von katalytischen Piperidinacetat bei Temperaturen von -10° C bis 100° C herstellt.

Als Lösungsmittel kommen vorzugsweise aliphatische Alkohole wie Methanol, Ethanol und/oder Isopropanol zur Anwendung. Die Umsetzungstemperaturen betragen bevorzugt 20-60° C.

Der Katalysator wird bevorzugt in Mengen von 0,01 bis 0,7 Mol, besonders bevorzugt von 0,02 - 0,2 Mol, insbesondere von 0,04-0,2 Mol pro Mol Ketocarbonsäureester zugesetzt.

Pro Mol Ketocarbonsäurester der Formel IV können bevorzugt 1 bis 2 Mol, insbesondere 1 Mol Aldehyd eingesetzt werden.

Bevorzugt bedeutet in der Formel I

R einen 2- oder 3-Nitrophenylrest, einen 2- oder 3-Chlorphenylrest oder einen 2,3-Dichlorphenylrest und

$R_1$ Methyl, Ethyl, Propyl, Isopropyl, Isobutyl oder einen Propoxyethylrest.

Die Umsetzung der Ylidenverbindung der Formel II mit der Enaminverbindung der Formel III erfolgt bei Temperaturen von -10 bis 130° C, vorzugsweise von 50 bis 100° C.

Pro Mol Ylidenverbindung können bevorzugt 1 bis 1,5 Mol, besonders bevorzugt 1 bis 1,3, insbesondere 1 bis 1,2 Mol der Enaminverbindung eingesetzt werden.

Nach einer besonderen Ausführungsform verbleibt die kristalline Ylidenverbindung im Reaktionsgefäß und wird direkt mit der Enaminverbindung umgesetzt.

Es ist als ausgesprochen überraschend zu bezeichnen, daß gemäß der erfindungsgemäßen Umsetzung die Yliden-3-ketocarbonsäureester in Gegenwart des genannten Katalysators in großer Reinheit und exzellenter Ausbeute entstehen und sich sehr gut isolieren lassen.

Weiterhin ist es als überraschend zu bezeichnen, daß auf die beschriebene Art und Weise die 1,4-Dihydropyridinverbindungen in so großer Reinheit entstehen und isoliert werden können. Sie enthalten - ohne weiteres Reinigungsverfahren - keine Nebenprodukte.

Das erfindungsgemäße Verfahren weist eine Reihe von Vorteilen auf.

So ist die Ausbeute höher als nach den bekannten Verfahren und das isolierte Produkt braucht keinen weiteren Reinigungsschritten unterworfen zu werden.

Verwendet man o-Nitrobenzaldehyd, Acetessigsäuremethylester und 3-Aminocrotonsäuremethylester als Ausgangsstoffe, so kann der Reaktionsablauf durch das folgende Formelschema wiedergegeben werden:

A)

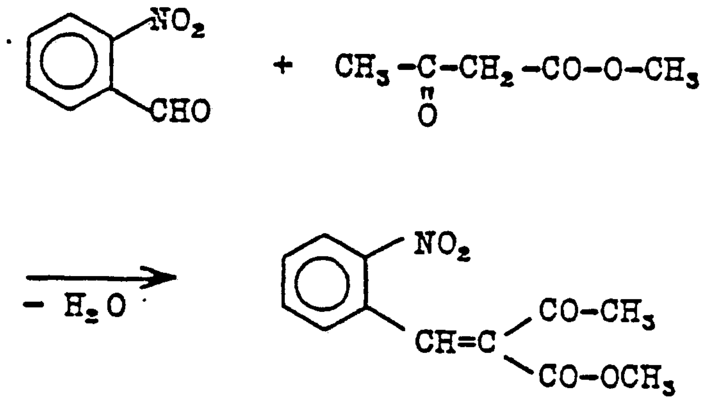


B)

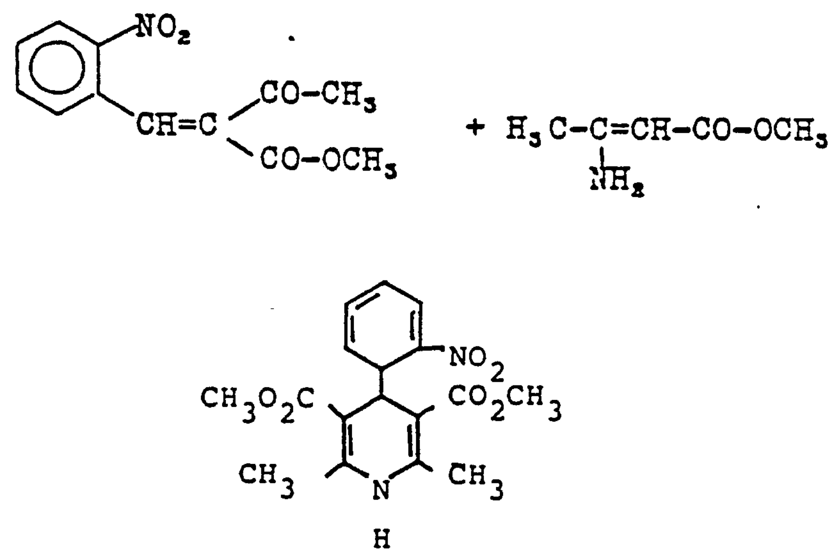

Die Erfindung sei anhand folgender Beispiele erläutert:

a) Ylidencarbonsäureester

Unter Rühren gibt man bei Raumtemperatur zu 650 ml Isopropanol in der folgenden Reihenfolge 116 g (1 Mol) Acetessigsäuremethylester, 151 g (1 mol) 2-Nitrobenzaldehyd, 2,4 g (0,04 Mol) Eisessig und 3,4 g (0,04 Mol) Piperidin.

Man erwärmt auf 40°C und läßt 15 Minuten bei dieser Temperatur.

Dann kühlt man auf 20°C ab und rührt 16 Stunden nach. Danach kühlt man auf 0°C ab und rührt bei dieser Temperatur 1 Stunde nach. Dann wird die überstehende Lösung abgesaugt, das Kristallisat mit 130 ml eiskaltem Isopropanol abgeschleudert und dieses abgesaugt.

Der entstandene 2-(2'-Nitrobenzyliden)-acetessigsäuremethylester wird im gleichen Gefäß sofort umgesetzt, wie in b) beschrieben.

Isoliert man den 2-(2'-Nitrobenzyliden)-acetessigsäuremethylester und trocknet ihn, erhält man 241,7 g eines gelben Kristallisats (97 % der theoret. Ausbeute) vom Schmp. 99-101°C.

Setzt man statt 0,04 Mol Piperidinacetat andere Katalysatormengen ein, erhält man folgende Ausbeuten:

| Menge | Ausbeute (%) |
|---|---|
| 0,08 Mol | 96,5 |
| 0,16 Mol | 97,6 |
| 0,5 Mol | 99,9 |

b) Zum nach a) hergestellten, isopropanolfeuchten 2,-(2'-Nitrobenzyliden)-acetessigsäuremethylester gibt man 750 ml Methanol und 111,5 g (0,97 Mol) 3-Aminocrotonsäuremethylester. Man erhitzt zum Rückfluß (ca. 65°C) und läßt 36 Stunden bei dieser Temperatur.

Nach dem Abkühlen auf 0°C wird das entstandene Kristallisat isoliert und mit 130 ml Methanol und 500 ml Wasser gewaschen und trockengesaugt.

Man erhält 302 g (0,87 Mol) 4-(2-Nitrophenyl)-2,6-dimethyl-3,5-dicarbmethoxy-1,4-dihydropyridin vom Schmelzpunkt 171-175°C (87 % der theoret. Ausbeute).

Die Dünnschichtchromatographie auf Merck-Kieselgelfertigplatten (Laufmittel: Chloroform: Aceton: Petrolether = 3 : 2 : 5) zeigt keine sichtbaren Nebenprodukte.

**Vergleichsversuch**

Beispiel 1 der US-PS 3 485 847 wurde nachgearbeitet und 4- (2-Nitrophenyl) -2,6-dimethyl-3,5-dicarboethoxy-1,4-dihydropyridin wie folgt erhalten:

| | |
|---|---|
| Ausbeute (%) | 80 |
| Nebenprodukte (Anzahl) | 7 |
| Farbe | rotbraun |

Die gleiche Verbindung wurde nach obigem, erfindungsgemäßen Verfahren a und b hergestellt. Folgende entsprechende Werte wurden erhalten:

| | |
|---|---|
| Ausbeute (%) | 85 |
| Nebenprodukte (Anzahl) | keine |
| Farbe | gelb |

**Patentansprüche**

1. Verfahren zur Herstellung von symmetrischen 1,4-Dihydropyridinen der Formel I

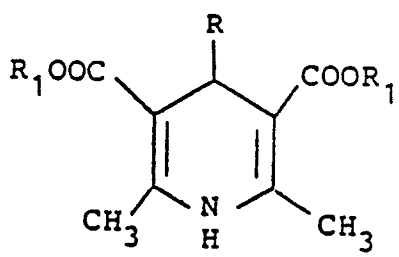

(I)

in der

R einen Phenylrest darstellt, der gegebenenfalls ein- oder zweifach substituiert ist durch Nitro und/oder Chlor und

$R_1$ einen $C_1$-$C_4$-Alkylrest darstellt, der gegebenenfalls durch eine $C_1$-$C_4$-Alkoxygruppe substituiert ist, durch Umsetzung einer Ylidenverbindung der Formel II

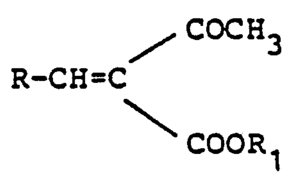

(II)

mit einer Enaminverbindung der Formel III

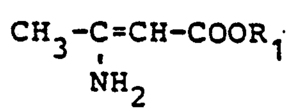

(III)

dadurch gekennzeichnet, daß man die Ylidenverbindung der Formel II durch Reaktion eines Ketocarbonsäureesters der Formel IV

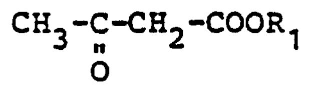

(IV)

mit einen Aldehyd der Formel RCHO in einen Lösungsmittel in Gegenwart von katalytischen Mengen von Piperidinacetat bei Temperaturen von -10° C und bis 100° C herstellt.

2. Verfahren gemäß Anspruch 1, dadurch gekennzeichnet, daß man die Umsetzung bei 20-60° C durchführt.

3. Verfahren gemäß Anspruch 1, dadurch gekennzeichnet, daß man pro Mol eingesetzten Ketocarbonsäureestern 0,01 bis 0,7 Mol an Katalysator einsetzt.

4. Verfahren gemäß Anspruch 1, dadurch gekennzeichnet, daß man pro Mol eingesetzten Ketocarbonsäureesters 0,02 bis 0,2 Mol an Katalysator einsetzt.

5. Verfahren gemäß Anspruch 1, dadurch gekennzeichnet, daß man pro Mol eingesetzten Ketocarbonsäureesters 0,04 bis 0,2 Mol an Katalysator einsetzt.

6. Verfahren gemäß Anspruch 1, dadurch gekennzeichnet, daß pro Mol Ketocarbonsäureester 1 bis 2 Mol Aldehyd eingesetzt wird.

7. Verfahren gemäß Anspruch 1, dadurch gekennzeichnet, daß pro Mol Ketocarbonsäureester 1 Mol Aldehyd eingesetzt wird.

**Claims**

1. Process for the preparation of symmetrical 1,4-dihydropyridines of the formula I

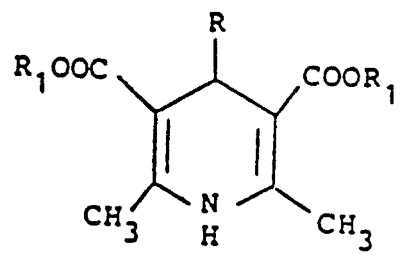

(I)

in which
R represents a phenyl radical which is optionally mono- or disubstituted by nitro and/or chlorine,
and
$R_1$ represents a $C_1$-$C_4$-alkyl radical which is optionally substituted by a $C_1$-$C_4$-alkoxy group, by reaction of an ylidene compound of the formula II

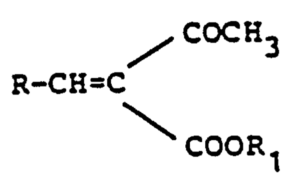

(II)

with an enamine compound of the formula III

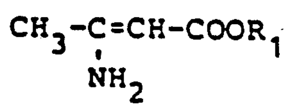

(III)

characterised in that the ylidene compound of the formula II is prepared by reaction of a ketocarboxylic ester of the formula IV

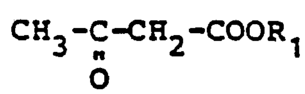

(IV)

with an aldehyde of the formula RCHO in a solvent, in the presence of catalytic amounts of piperidine acetate, at temperatures from -10° C and up to 100° C.

2. Process according to Claim 1, characterised in that the reaction is carried out at 20-60° C.

3. Process according to Claim 1, characterised in that 0.01 to 0.7 mol of catalyst is employed per mol of ketocarboxylic esters employed.

4. Process according to Claim 1, characterised in that 0.02 to 0.2 mol of catalyst is employed per mol of ketocarboxylic esters employed.

5. Process according to Claim 1, characterised in that 0.04 to 0.2 mol of catalyst is employed per mol of ketocarboxylic ester employed.

6. Process according to Claim 1, characterised in that 1 to 2 mol of aldehyde is employed per mol of ketocarboxylic ester.

7. Process according to Claim 1, characterised in that 1 mol of aldehyde is employed per mol of ketocarboxylic ester.

**Revendications**

1. Procédé de fabrication de 1,4-dihydropyridines symétriques de formule I:

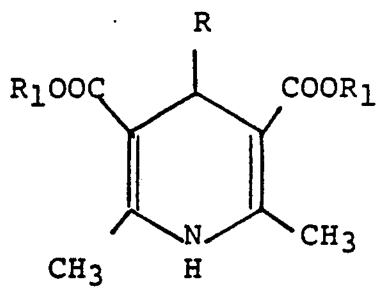

(I)

dans laquelle

R représente un radical phényle qui est éventuellement substitué une ou deux fois par un nitro et/ou chloro et

$R_1$ un radical alcoyle en $C_1$-$C_4$ qui est éventuellement substitué par un groupe alcoxy en $C_1$-$C_4$,

par réaction d'un composé ylidène de formule II

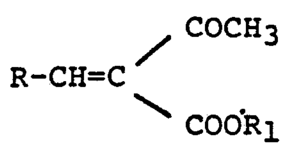

(II)

avec un composé ènamine dè formule III

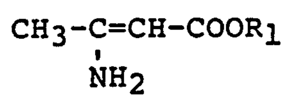

(III)

caractérisé en ce qu'on prépare le composé ylidène de formule II par réaction d'un ester cétocarboxylique de formule IV

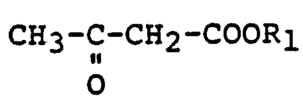

(IV)

avec une aldéhyde de formule RCHO dans un solvant en présence de quantités catalytiques d'acétate de pipéridine à des températures de -10° C à 100° C.

2. Procédé selon la revendication 1, caractérisé en ce qu'on exécute la réaction à 20 - 60° C.

3. Procédé selon la revendication 1, caractérisé en ce qu'on utilise par mole d'esters cétocarboxyliques employés 0,01 à 0,7 mole de catalyseur.

4. Procédé selon la revendication 1, caractérisé en ce qu'on utilise par mole d'ester cétocarboxylique employé 0,02 à 0,2 mole de catalyseur.

5. Procédé selon la revendication 1, caractérisé en ce qu'on utilise par mole d'ester cétocarboxylique

employé 0,04 à 0,2 mole de catalyseur.

6. Procédé selon la revendication 1, caractérisé en ce qu'on utilise par mole d'ester cétocarboxylique 1 à 2 moles d'aldéhyde.

7. Procédé selon la revendication 1, caractérisé en ce qu'on utilise par mole d'ester cétocarboxylique 1 mole d'aldéhyde.